(19) Europäisches Patentamt
European Patent Office
Office européen des brevets

(11) **EP 2 033 570 B1**

(12) **EUROPEAN PATENT SPECIFICATION**

(45) Date of publication and mention
of the grant of the patent:
**24.04.2013 Bulletin 2013/17**

(51) Int Cl.:
*A61B 1/05* (2006.01)        *A61B 1/06* (2006.01)
*A61B 1/273* (2006.01)

(21) Application number: **08010175.1**

(22) Date of filing: **04.06.2008**

(54) **Capsule endoscope**

Kapselendoskop

Endoscope de type capsule

(84) Designated Contracting States:
**DE FR GB**

(30) Priority: **06.09.2007   JP 2007231362**

(43) Date of publication of application:
**11.03.2009   Bulletin 2009/11**

(73) Proprietor: **Olympus Medical Systems Corp.
Tokyo 151-0072 (JP)**

(72) Inventors:
• **Orihara, Tatsuya
Tokyo 151-0072 (JP)**

• **Fukuhori, Hitoshi
Tokyo 151-0072 (JP)**

(74) Representative: **von Hellfeld, Axel
Wuesthoff & Wuesthoff
Patent- und Rechtsanwälte
Schweigerstrasse 2
81541 München (DE)**

(56) References cited:
**EP-A- 1 790 280        WO-A-01/50941
US-A1- 2006 170 328      US-A1- 2007 055 105
US-B1- 6 261 226**

**Description**

BACKGROUND OF THE INVENTION

The present invention relates generally to a capsule endoscope, and more particularly to an optimum structure for wide-field observation by capsule endoscopes.

Unlike conventional endoscopes, current capsule endoscopes have not a function of implementing in-vivo scans in any desired field direction; as compared with an endoscope having the same field range, a capsule endoscope would have a blind spot in a range incapable of changing the field of view, resulting in an increased probability of some oversight of lesions.

For this reason, the capsule endoscope having no function of implementing scans over the field range is designed to have a wide-angle, ahead-and-behind binocular imaging system (Patent Publication 1) so that any blind spot can be eliminated to stave off the oversight of lesions; the binocular imaging system is said to be a function of urgent need.

However, making the optical system a wide-angle arrangement leads to use of a number of lenses, resulting in total length increases and cost rises; there is a mounting demand toward achieving that wide-angle arrangement with as much reduced lens counts as possible (Patent Publication 2).

When a similar illumination system as used heretofore is employed while the range of field of an objective system is just widened, the range of field widened by the wide-angle arrangement is less brightly illuminated, possibly ending up with a drop of the rate of spotting lesions.

To take advantage of improvements in the performance due to the wide-angle arrangement for the imaging system, it is simultaneously necessary to distribute illumination light over a wider range (Patent Publications 3 and 4).

Patent Publication 1
Published Translation 2005-503182
Patent Publication 2
JP(A)2005-80713
Patent Publication 3
Internal Publication WO2004/096029
Patent Publication 4
JP(A)2004-275542

Document EP 1 790 280 A1 discloses a capsule endoscope having an imaging device with front and rear transparent optical domes. The imaging device comprises on each side an imager, a lens holder and illumination sources, which are arranged on a planar end of a frame member. Further, the lens holders contain various optical components such as optical lenses for focussing light on the imagers.

Document US 2007/0055105 A1 discloses a capsule-type endoscope comprising an objective optical system covered by a transparent cover. Illumination sections are arranged around the objective optical system on tilting bases. Further, the tilting bases are provided on the front side of an illumination substrate configured into a doughnut-shape such that the illumination sections make a preset angle with the optical axis of the objective optical system.

Further technological background can be found in US 6,261,226 B1, US 2006/0170328 A1 and WO 01/50941 A2.

In view of such situations with the prior art as described, the present invention has for its object to provide a capsule endoscope layout capable of achieving a small-format, wide-angle, wide light-distribution arrangement with limited variations.

SUMMARY OF THE INVENTION

According to the invention, the aforesaid object is accomplishable by the provision of a capsule endoscope according to independent claim 1. Further features of the invention are described in the dependent claims.

The invention provides a capsule endoscope which comprises an objective lens, a transparent dome to cover the object side of said objective lens, and light emitter devices located around the outer periphery of said objective lens, characterized in that:

there are two objective lenses provided in an ahead and behind direction of a housing, there is a transparent dome provided to cover the object sides of said objective lenses, there are light emitter devices provided around the outer peripheries of said objective lenses, and the light emitter devices provided ahead and behind the housing are located at a position set back from the end of each objective lens and inclined outward with respect to the center axis of each objective lens so that light distributed from the light emitter devices located ahead and behind the housing intersects around the housing.

To locate two wide-angle objective optical systems ahead and behind into a binocular arrangement for viewing images all around, the light emitter devices located ahead and behind should be such that the distributed light intersects ahead and behind and around them, thereby getting rid of portions that illumination light does not arrive at.

In the invention, the aforesaid objective lens has a field range of 140° or greater and satisfies the following condition (1):

$$0° < \theta \leqq 60° \qquad\qquad (1)$$

where $\theta$ is an angle that the center axis of each light emitter device in the radial direction makes with the center axis of the objective lens.

Preferably in the invention, the aforesaid objective lens has a three lenses arrangement comprising, in order from its object side, a meniscus lens having negative refracting power and convex on its object side, a lens having negative refracting power, a stop and a lens having positive refracting power.

The aforesaid light emitter device may be made up of a light emitting diode (LED), and an electroluminescent device (EL).

The former is bright and less costly. Attached to the flexible substrate, the latter is thin and may be attached even in narrow space, and is of fast response as well.

It is also desired to satisfy the following condition (2):

$$Ra > L \qquad\qquad (2)$$

where Ra is the radius of curvature of said object lens surface on the aforesaid transparent dome side, and L is the distance of the apex of the aforesaid objective lens surface on the aforesaid transparent dome side to the surface located in, and nearest to the object side of the aforesaid objective lens.

Thus, even when the objective lens makes its way into the transparent dome by way of a wide-angle objective system, the narrowing of the field does not occur so that the length of the whole capsule endoscope can be curtailed, making much contribution to easing off burdens on patients.

Further, the invention provides a capsule endoscope wherein the aforesaid light emitter devices are located such that when light is emitted out of each light emitter device onto a spherical object, light emitted out of the light emitter devices located ahead and behind the housing and having an intensity of 10% or greater intersects, wherein it is assumed that the intensity of light emitted in the direction of the center axis of each light emitter device in the radial direction is 100%.

Yet further, the invention provides a capsule endoscope that satisfies the following condition (3):

$$(N/2) / \tan (\beta-90°) \geqq (M/2) / \tan (\alpha+ \theta-90°) \qquad\qquad (3)$$

where N is a longitudinal distance between the centers of the light emitter devices located ahead and behind the housing; M is a longitudinal distance between the centers of the ends of the objective lenses located ahead and behind the housing; $\alpha$ is an angle with respect to the center axis at which there is a 10% intensity with respect to the intensity of light given out in the direction of the center axis of the light emitter device in the radial direction; $\beta$ is a half the angle of field of the objective lenses; and $\theta$ is an angle that the center axes of the light emitter devices in the radial direction makes with the center axes of the objective lenses.

According to the invention, the objective lenses are configured into a wide-angle arrangement, and the application of this arrangement to an illumination system layout for a conventional capsule endoscope would render the brightness of its periphery to be less sufficient, working against observations. However, the light emitter devices are inclined and located around the optical system so that it is possible to achieve a wide light-distribution illumination system compatible even with a wide-angle optical system, resulting in improvements in screening capability due to a wide-angle-of-field, wide light-distribution arrangement.

By locating two objective lenses each having an angle of field of 140° or greater in the ahead-and-behind direction to set up a binocular arrangement capable of viewing images nearly all around, it is possible to make substantial elimination of any blind spot. It is thus possible to get rid of blind spots even with a capsule endoscope having no function of changing the field direction freely and, hence, make improvements in screening capability.

Still other objects and advantages of the invention will in part be obvious and will in part be apparent from the specification.

The invention accordingly comprises the features of construction, combinations of elements, and arrangement of parts which will be exemplified in the construction hereinafter set forth, and the scope of the invention will be indicated in the claims.

BRIEF DESCRIPTION OF THE DRAWINGS

Fig. 1 is illustrative in section of the construction of an end of the inventive capsule endoscope.

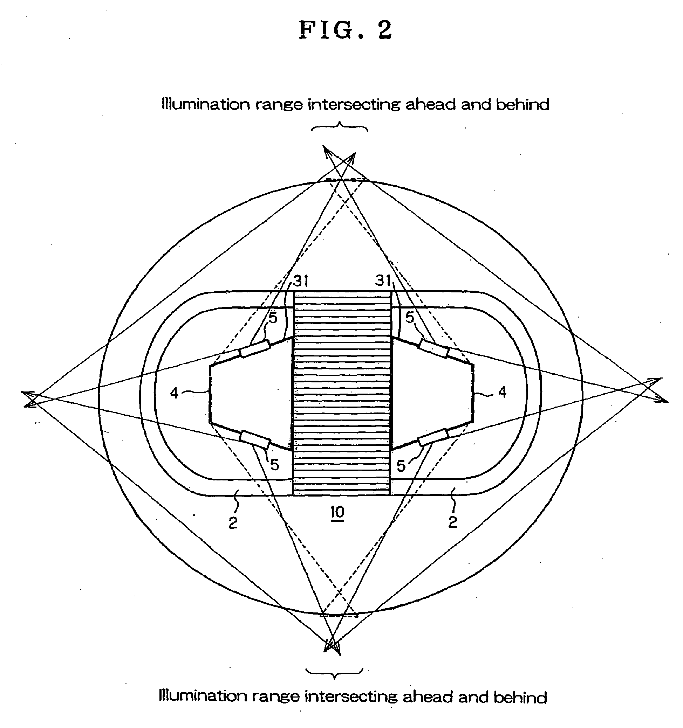

Fig. 2 is illustrative in section of a binocular type capsule endoscope wherein such end structures as shown in Fig. 1 are located ahead and behind.

Fig. 3 is illustrative in schematic of how the small intestine is observed and diagnosed inside by the capsule endoscope of Fig. 2.

Fig. 4(a) is illustrative in section of one exemplary construction of an end of the inventive capsule endoscope and Fig. 4(b) is a front view of the holder frame.

Fig. 5 is illustrative of one exemplary angle that the center axis of the light emitter device in the radial direction makes with the center axis of the objective lens, and how light is distributed then.

Fig. 6 is illustrative of how light is distributed from one exemplary light emitter device.

Fig. 7 is illustrative in schematic of the inventive capsule endoscope using the light emitter device of Fig. 6.

Fig. 8 is illustrative in section of an end structure of a prior art capsule endoscope.

Fig. 9 is illustrative in section of a prior art binocular type capsule endoscope.

DESCRIPTION OF THE PREFERRED EMBODIMENTS

Examples of the inventive capsule endoscope are now explained with reference to the accompanying drawings.

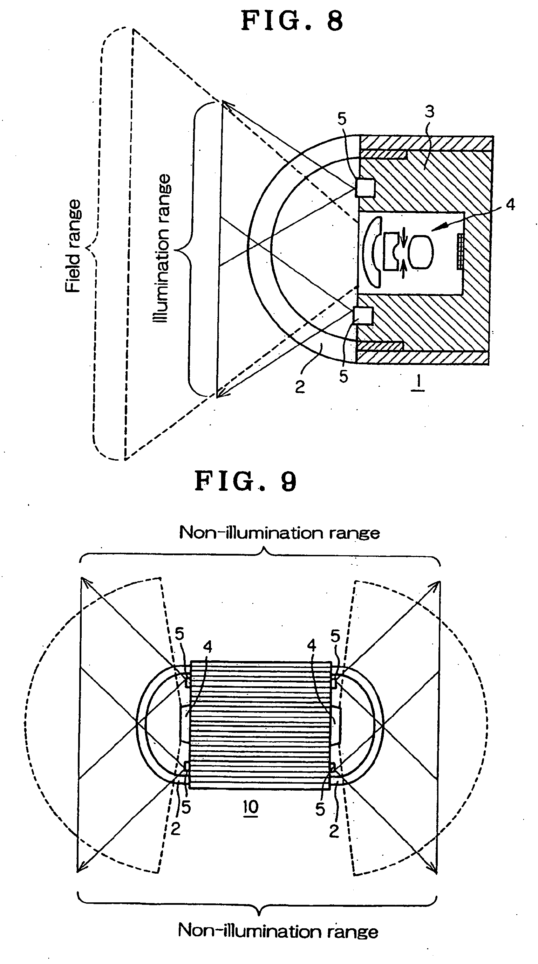

Referring to Fig. 8 that is illustrative of an end structure of a conventional capsule endoscope 1, a transparent, semispherical dome 2 is located over the end of the capsule endoscope 1; within the capsule endoscope 1 there is an objective lens 4 attached to the center of a frame member 3; and at a planar end of the frame member 3 around the objective lens 4 there are a plurality of light emitter devices 5 located symmetrically about the center axis, wherein each light emitter device comprises a light emitter diode (LED) or electroluminescent device (EL). In such arrangement, the field range (indicated by a broken line) of the objective lens 4 is included in the illumination range (indicated by a solid line) of the light emitter devices 5.

As end structures of such arrangement are located ahead and behind into a binocular type capsule endoscope 10, it causes a non-illumination range (area) to appear between the illumination ranges of both light emitter devices 5; even when the field range (broken line) of each objective lens 4 is widened, that non-illumination range renders brightness insufficient, resulting a drop of the rate of spotting lesions.

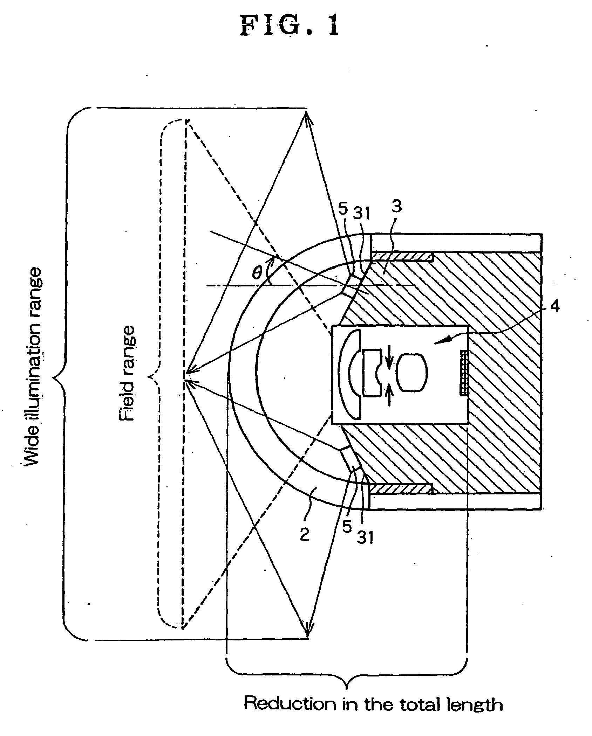

Figure 1 shows a capsule endoscope 1 wherein an end surface 31 of a frame member 3 around an objective lens 4 is configured as a conical or pyramidal shape such that an end of the centrally located objective lens 4 juts out in a dome 2 and positions of a plurality of light emitter devices 5 located around there are set back from an end of the objective lens 4, and the light emitter devices 5 are attached to that conical or pyramidal end surface 31 such that they direct outward obliquely with respect to the center axis of the objective lens 4. The range of illumination by the symmetrically located light emitter devices 5 grows wide and, with this, the field range of the objective lens 4 grows wide, resulting in a wider field range.

The field range of the objective lens 4 here is desirously 140° or greater. And in letting $\theta$ stand for an angle that the center axis of each light emitter device 5 in the radial direction makes with the center axis of the objective lens 4, it is desirous to satisfy the following condition.

$$0° < \theta \leqq 60° \qquad\qquad (1)$$

At the lower limit of 0° to condition (1), the same thing as in the prior art of Fig. 8 takes place: the endoscope runs short of illumination at off-axis sites with a drop of the rate of spotting lesions. As the upper limit of 60° is exceeded, on the contrary, the endoscope is likely to run short of illumination on the center axis.

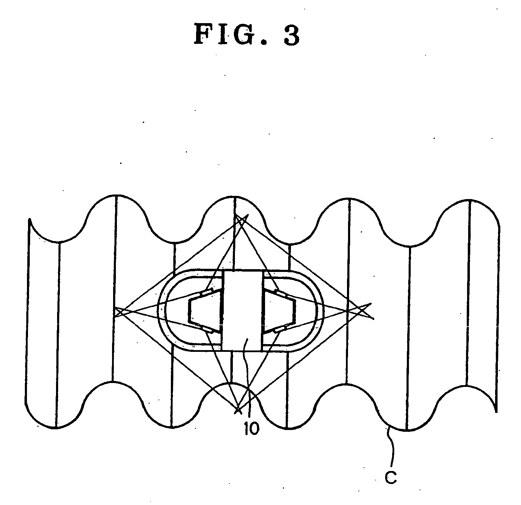

Referring now to a binocular type capsule endoscope 10 comprising such end structures as shown in Fig. 1 located ahead and behind, the illumination ranges by a plurality of light emitter devices 5 at the respective ends are set wider than 180° such that the ranges (illumination ranges) of light distributed from the light emitter devices 5 located ahead and behind intersect mutually (of course, the ranges of light distributed from a plurality of light emitter devices 5 located at the respective ends intersect mutually, too). This makes it possible to illuminate almost all around the capsule endoscope 10 so that, as shown schematically shown in Fig. 3, for instance, the small intestine C can be observed and diagnosed with no blind spot yet with a very low probability of losing sight of lesions.

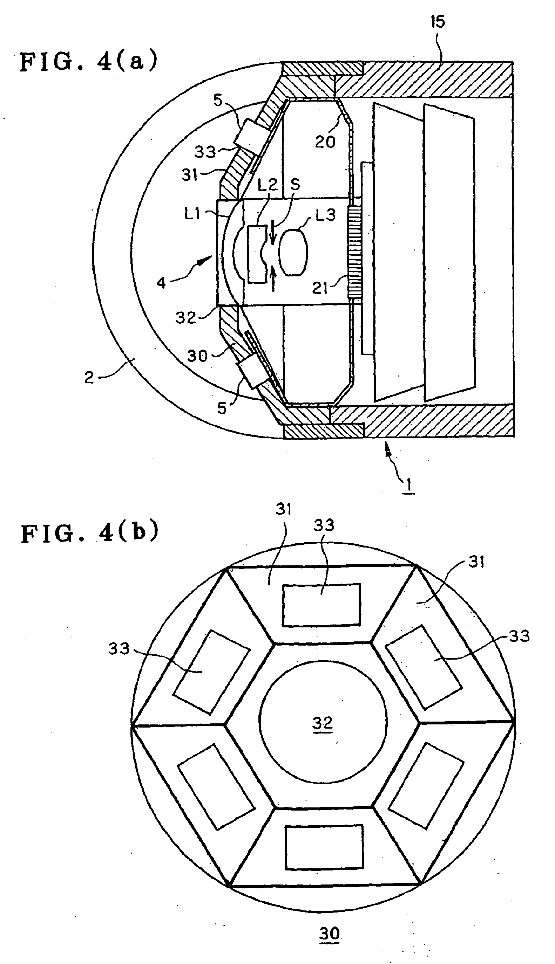

Fig. 4(a) is illustrative in section of one exemplary construction of one end of the capsule endoscope 1. At the end of a cylindrical housing 15 of the capsule endoscope, there is a holder frame 30 fixed, whose front view is presented in Fig. 4(b), and over that, a transparent, semispherical dome 2 is covered to form the end of the capsule endoscope 1. The holder frame 30 is formed of a sheet metal of hexagonal pyramid shape having in the center of an apex surface an opening 32 into which an objective lens 4 is fitted, and an opening 33 for fixing a light emitter device 5 is provided in one each side of the hexagonal pyramid. A lens barrel of the objective lens 4 is coaxially fixed in the opening 32 in the apex surface of the holder frame 30 of hexagonal pyramid shape, and a flexible substrate 20 having light emitter devices 5 at a given interval on its front surface is pressed against and fixed to the inside surface (back surface) of the holder frame 30, so that one each light emitter device 5 is inserted through and fixed in the opening 33 in one each side of the holder frame 30 from within the holder frame 30.

As set forth typically in Patent Publication 2, the objective lens 4 is of a three lenses type that is made up of, in order from its object side, a meniscus lens L1 having negative refracting power and convex on its object side, a lens L2 having negative refracting power, a stop S and a lens L3 having positive refracting power, so that the angle of field to be viewed can be set to 140° or greater, and preferably 180° or greater with no blind spot, leading to much less chances of losing sight of lesions. And an imaging device 21 such as CCD is located on the image plane of the objective lens 4 for connection to the flexible substrate 20.

Thus, by application to the end structure of the capsule endoscope 1 of the holder frame 30 capable of precisely determining the positions of location of the objective lens 4 and light emitter devices 5, it is possible to achieve a structure capable of holding the imaging system and the illumination system as an integral piece, thereby determining the location of the objective lens 4 and light emitter devices 5 with high precision. The light emitter devices 5 are mounted on the flexible substrate 20 that enables the directions of the devices to be freely determined, but without any holder structure, however, the location of the light emitter devices 5 would get erratic, possibly causing variations in light distribution, and flares. However, if, as described above, the integral-piece holder frame 30 is used which allows the light emitter devices 5 attached to the flexible substrate 20 to be fixed in place and the positions of the objective lens 4 and light emitter devices 5 to be determined with high precision, it is then possible to reduce the variations in light distribution and unwanted light, and improve assembly capabilities as well.

With the inventive capsule endoscope 1, the light emitter devices 5 are attached to the inclined sides of the holder frame 30 around the objective lens 4; the objective lens 4 is positioned jutting out in the transparent semispherical dome 2. It is thus possible just only to achieve a wide-field, wide light-distribution arrangement but also to reduce dead space in the dome 2 and curtail the length of the whole of the capsule endoscope 1 by the amount of jutting of the objective lens 4 into the dome 2. A reduction in the total length of the capsule endoscope 1 helps reduce burdens on patients and take hold of safety. To this end, it is desired to satisfy the following condition.

$$Ra > L \qquad\qquad (2)$$

Here Ra is the radius of curvature of the surface of the objective lens 4 on the transparent dome 2 side, and L is the distance from the apex of the surface of the objective lens 4 on the transparent dome 2 side to the surface located in, and nearest to the object side of, the objective lens (the object-side surface of the meniscus lens L1).

Beyond of the range of condition (2), the aforesaid effect on reductions of the total length is not obtainable.

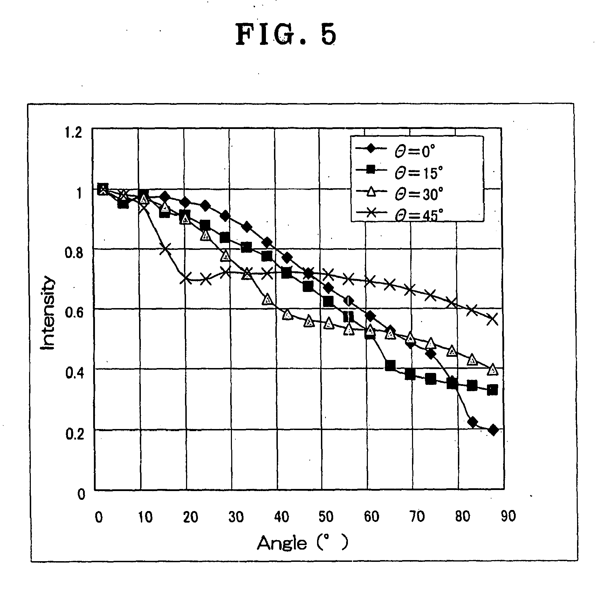

Referring then to Fig. 5, an example of the angle θ of the center axis of the light emitter device 5 in the radial direction with respect to the center axis of the objective lens 4 is shown together with light distributions in that case. A reference light distribution for each light emitter device 5 is obtained at θ=0, and light distributions at angles of 0 to 90° with the center axis of the objective lens 4 located at the angle θ of 15°, 30°, and 45° are drawn in Fig. 5. At the angle of location θ=45°, even the periphery of the objective lens 4 at 90° with respect to the center axis of the objective lens 4 is going to be brightly illuminated.

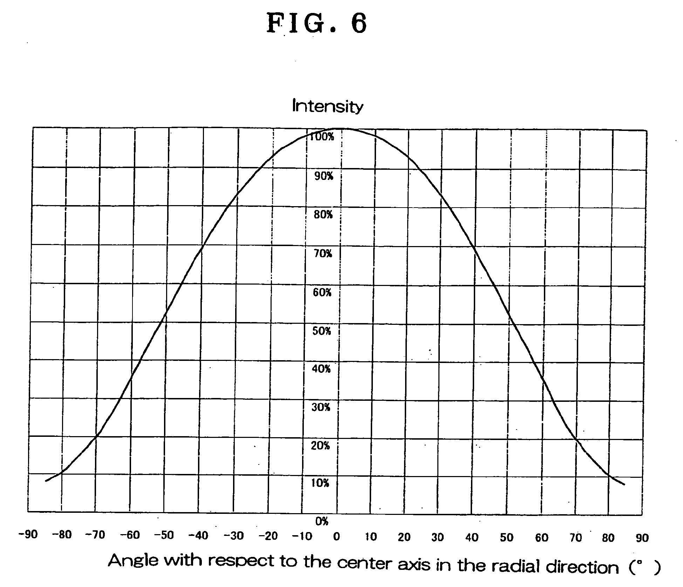

Fig. 6 is illustrative of light distributions for one example of a spherical form of light emitter device used here. The angle with respect to the center axis at which there is a 10% intensity with respect to the intensity of light given out in the direction of the center axis of the light emitter device in the radial direction is 80°.

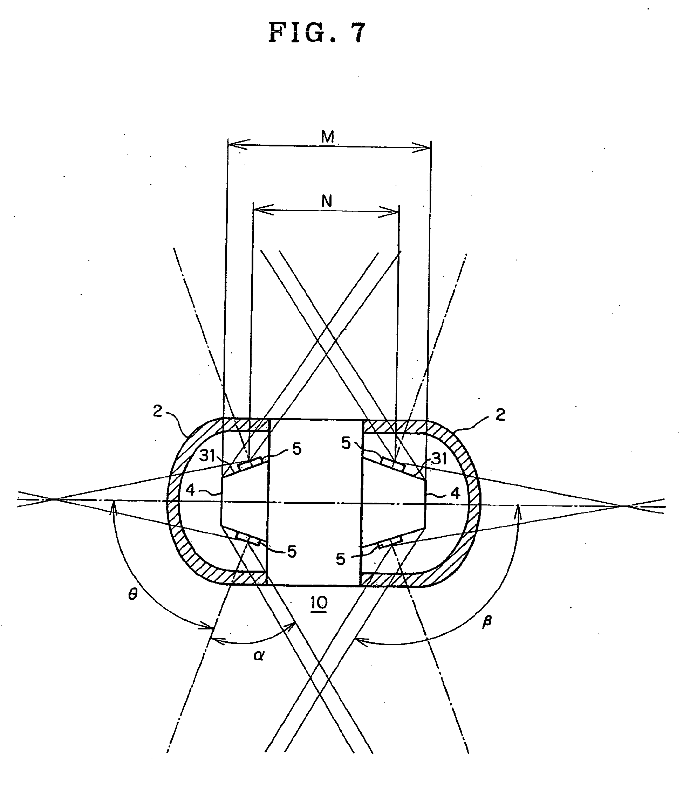

Fig. 7 is illustrative in schematic of the inventive capsule endoscope 10 with which that light emitter device 5 is used. A longitudinal distance N between the centers of light emitter devices 5 located ahead and behind is 10 mm; a longitudinal distance M between the centers of the ends of objective lenses 4 located ahead and behind is 11 mm; an angle α with respect to the center axis at which there is a 10% intensity with respect to the intensity of light given out in the direction of the center axis of the light emitter device 5 in the radial direction is 80°; an angle θ that the center axis of the light emitter device 5 in the radial direction makes with the center axis of the objective lens 4 is 35°; and a half β the angle of field of the objective lens 4 is 110°. That is,

$$(N/2)/\tan(\beta-90°)=13.7$$

$$(M/2)/\tan(\alpha+\theta-90°)=11.8$$

of which $(N/2)/\tan(\beta-90°)=13.7$ is larger.

$$(N/2)/\tan(\beta-90°)\geqq(M/2)/\tan(\alpha+\theta-90°) \qquad (3)$$

By satisfying condition (3), the light given out of the light emitter-device 5 falling within the field of view is supposed to have an intensity of 10% or greater; even when the objective lens has a wide-angle field, a bright image can be viewed as far as its periphery.

While the inventive capsule endoscope has been described with reference to its examples, it is appreciated that the invention is never limited to them: various modifications may be achievable.

## Claims

1. A capsule endoscope (1) which comprises an objective lens (4), a transparent dome (2) to cover the object side of said objective lens (4), and light emitter devices (5) located around the outer periphery of said objective lens (4), wherein:

   there are first and second objective lenses (4) provided in an ahead and behind direction of a housing (15), respectively, there is a transparent dome (2) provided to the object sides of said objective lenses (4), there are light emitter devices (5) provided around the outer peripheries of said objective lenses (4), and the light emitter devices (5) located ahead and behind the housing (15) are located at a position set back from the end of each objective lens (4),
   wherein the light emitter devices (5) are inclined outward, and
   wherein each objective lens (4) has a field range of 140° or greater;

   **characterized in that** the capsule endoscope (1) satisfies the following conditions (1, 2):

$$0° < \theta \leq 60° \qquad (1)$$

   so that light distributed from the light emitter devices (5) located ahead and behind the housing (15) intersects around the housing (15); and

$$(N/2) / \tan (\beta - 90°) \geq (M/2) / \tan (\alpha + \theta - 90°) \qquad (2)$$

   wherein:

   N is a longitudinal distance between the centers of the light emitter devices (5) located ahead and behind the housing (15);
   M is a longitudinal distance between the centers of the ends of the objective lenses (4) located ahead and behind the housing (15);
   α is an angle with respect to the center axis at which there is a 10% intensity with respect to the intensity of light given out in the direction of the center axis of the light emitter device (5) in the radial direction;

β is a half the angle of field of the objective lenses (4); and

θ is an angle that the center axes of the light emitter devices (5) in the radial direction makes with the center axes of the objective lenses (4).

2. The capsule endoscope (1) according to claim 1, wherein each objective lens (4) comprises a three lenses arrangement comprising, in order from an object side thereof, a meniscus lens (L1) having negative refracting power and convex on an object side thereof, a lens (L2) having negative refracting power, a stop (S) and a lens (L3) having positive refracting power.

3. The capsule endoscope (1) according to claim 1, **characterized in that** each light emitter device (5) comprises a light-emitting diode (LED).

4. The capsule endoscope (1) according to claim 1, **characterized in that** each light emitter device (5) comprises an electroluminescent device (EL).

**Patentansprüche**

1. Kapselendoskop (1), das eine Objektivlinse (4), eine transparente Kuppel (2) zum Abdecken der Objektseite der Objektivlinse (4) und Lichtsendeeinrichtungen (5) aufweist, die um den Außenumfang der Objektivlinse (4) herum angeordnet sind, wobei:

es erste und zweite Objektivlinsen (4) gibt, die jeweils in einer Richtung vor und hinter einem Gehäuse (15) vorgesehen sind, es eine transparente Kuppel (2) gibt, die zu den Objektseiten der Objektivlinsen (4) hin vorgesehen ist, es Lichtsendeeinrichtungen (5) gibt, die um die Außenumfänge der Objektivlinsen (4) herum vorgesehen sind, und die vor und hinter dem Gehäuse (15) angeordneten Lichtsendeeinrichtungen (5) in einer Position angeordnet sind, die vom Ende jeder Objektivlinse (4) zurückgesetzt ist,

wobei die Lichtsendeeinrichtungen (5) nach außen geneigt sind, und

wobei jede Objektivlinse (4) einen Feldbereich von 140° oder mehr aufweist;

**dadurch gekennzeichnet, dass** das Kapselendoskop (1) die folgenden Bedingungen (1, 2) erfüllt:

$$0° < θ ≤ 60° \qquad\qquad\qquad (1)$$

sodass Licht, das von den vor und hinter dem Gehäuse (15) angeordneten Lichtsendeeinrichtungen (5) ausgestrahlt wird, sich um das Gehäuse (15) herum überschneidet; und

$$(N/2) / \tan(β - 90°) ≥ (M/2) / \tan(α + θ - 90°) \qquad (2)$$

wobei:

N ein Längsabstand zwischen den Mittelpunkten der vor und hinter dem Gehäuse (15) angeordneten Lichtsendeeinrichtungen (5) ist;

M ein Längsabstand zwischen den Mittelpunkten der Enden der vor und hinter dem Gehäuse (15) angeordneten Objektivlinsen (4) ist;

α ein Winkel zur Mittelachse ist, bei dem in radialer Richtung eine 10%-Intensität bezüglich der Lichtintensität vorhanden ist, die in der Richtung der Mittelachse der Lichtsendeeinrichtung (5) ausgegeben wird;

β der halbe Feldwinkel der Objektivlinsen (4) ist; und

θ ein Winkel ist, den die Mittelachsen der Lichtsendeeinrichtungen (5) in der radialen Richtung mit den Mittelachsen der Objektivlinsen (4) bilden.

2. Kapselendoskop (1) nach Anspruch 1, wobei jede Objektivlinse (4) eine Anordnung von drei Linsen umfasst, die von ihrer Objektseite folgende Reihenfolge aufweisen: eine Meniskuslinse (L1), die eine negative Brechkraft aufweist und an ihrer Objektseite konvex ist, eine Linse (L2) mit negativer Brechkraft, eine Blende (S) und eine Linse (L3) mit positiver Brechkraft.

3. Kapselendoskop (1) nach Anspruch 1, **dadurch gekennzeichnet, dass** jede Lichtsendeeinrichtung (5) eine Leuchtdiode (LED) aufweist.

4. Kapselendoskop (1) nach Anspruch 1, **dadurch gekennzeichnet, dass** jede Lichtsendeeinrichtung (5) eine Elektroluminiszenz-Einrichtung (EL) aufweist.

**Revendications**

1. Endoscope de type capsule (1) qui comprend un objectif (4), un dôme transparent (2) pour couvrir le côté objet dudit objectif (4), et des dispositifs d'émission de lumière (5) placés autour de la périphérie externe dudit objectif (4), dans lequel :

   des premier et deuxième objectifs (4) sont prévus vers l'avant et vers l'arrière d'un logement (15), respectivement, un dôme transparent (2) est prévu sur les côtés objet desdits objectifs (4), des dispositifs d'émission de lumière (5) sont prévus autour des périphéries externes desdits objectifs (4), et les dispositifs d'émission de lumière (5) placés vers l'avant et vers l'arrière du logement (15) sont placés au niveau d'une position établie à l'arrière depuis l'extrémité de chaque objectif (4),
   dans lequel les dispositifs d'émission de lumière (5) sont inclinés vers l'extérieur, et
   dans lequel chaque objectif (4) présente une plage de champs de 140° ou plus ;

   **caractérisé en ce que** l'endoscope de type capsule (1) satisfait les conditions suivantes (1, 2) :

$$0° < \theta \leq 60° \qquad\qquad (1)$$

   de sorte que les lumières émises depuis les dispositifs d'émission de lumière (5) placés vers l'avant et vers l'arrière du logement (15) se croisent autour du logement (15) ; et

$$(N/2)/\tan(\beta - 90°) \geq (M/2)/\tan(\alpha + \theta - 90°) \qquad (2)$$

   où :

   N représente une distance longitudinale entre les centres des dispositifs d'émission de lumière (5) placés vers l'avant et vers l'arrière du logement (15) ;
   M représente une distance longitudinale entre les centres des extrémités des objectifs (4) placés vers l'avant et vers l'arrière du logement (15) ;
   $\alpha$ représente un angle par rapport à l'axe central au niveau duquel il y a 10 % d'intensité par rapport à l'intensité de la lumière délivrée dans la direction de l'axe central du dispositif d'émission de lumière (5) dans la direction radiale ;
   $\beta$ représenté une moitié de l'angle de champ des objectifs (4) ; et
   $\theta$ représente un angle que les axes centraux des dispositifs d'émission de lumière (5) dans la direction radiale constituent avec les axes centraux des objectifs (4).

2. Endoscope de type capsule (1) selon la revendication 1, dans lequel chaque objectif (4) comprend une disposition de trois lentilles comprenant, dans l'ordre depuis un côté objet de celui-ci, une lentille convexo-concave (L1) présentant une puissance de réfraction négative et convexe sur un côté objet de celle-ci, une lentille (L2) présentant une puissance de réfraction négative, une butée (S) et une lentille (L3) présentant une puissance de réfraction positive.

3. Endoscope de type capsule (1) selon la revendication 1, **caractérisé en ce que** chaque dispositif d'émission de lumière (5) comprend une diode à émission de lumière (LED).

4. Endoscope de type capsule (1) selon la revendication 1, **caractérisé en ce que** chaque dispositif d'émission de lumière (5) comprend un dispositif électroluminescent (EL).

# FIG. 1

# FIG. 2

Illumination range intersecting ahead and behind

Illumination range intersecting ahead and behind

FIG. 3

FIG. 4(a)

FIG. 4(b)

FIG. 5

# FIG. 6

Intensity

Angle with respect to the center axis in the radial direction (° )

# FIG. 7

# FIG. 8

# FIG. 9

Non-illumination range

Non-illumination range

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005503182 A
- JP 2005080713 A
- WO 2004096029 A
- JP 2004275542 A
- EP 1790280 A1
- US 20070055105 A1
- US 6261226 B1
- US 20060170328 A1
- WO 0150941 A2